# EUROPÄISCHE PATENTSCHRIFT

(11) **EP 2 736 360 B1**
(45) Veröffentlichungstag und Bekanntmachung des Hinweises auf die Patenterteilung: **09.09.2015**
(21) Anmeldenummer: 12743375.3
(22) Anmeldetag: 24.07.2012
(51) Int. Cl.: A24F 47/00, A61M 15/06, A61M 11/04

(54) **INHALATORKOMPONENTE**
INHALER COMPONENT
COMPOSANT D'UN INHALATEUR

(30) Priorität: 27.07.2011 AT 10952011
(43) Veröffentlichungstag der Anmeldung: 04.06.2014
(60) Teilanmeldung: 15178588.8
(73) Patentinhaber: Batmark Limited, London WC2R 2PG (GB)
(72) Erfinder: BUCHBERGER, Helmut, A-4482 Ennsdorf (AT)
(74) Vertreter: Schrell, Andreas
(86) Internationale Anmeldenummer: PCT/EP2012/003103
(87) Internationale Veröffentlichungsnummer: WO 2013/013808

(56) Entgegenhaltungen:
- WO-A1-2010/045671
- CH-B1- 698 603
- GB-A- 191 125 575

## Beschreibung

Die Erfindung betrifft eine Inhalatorkomponente für die Bildung eines Dampf-Luft-Gemisches oder/und Kondensationsaerosols durch Verdampfung eines flüssigen Materials und gegebenenfalls Kondensation des gebildeten Dampfes, umfassend:
ein elektrisches Heizelement zur Verdampfung einer Portion des flüssigen Materials;
einen Docht mit einer Kapillarstruktur, welcher Docht mit dem Heizelement einen Verbund bildet und das Heizelement selbsttätig mit dem flüssigen Material versorgt;
eine Trägerplatte, vorzugsweise Leiterplatte, welche den Verbund trägt und auf welcher das Heizelement elektrisch kontaktiert ist;
einen zumindest zum Teil durch die Trägerplatte gebildeten Kapillarspalt zur selbsttätigen Versorgung des Verbundes mit dem flüssigen Material, indem ein Endabschnitt des Dochts in den Kapillarspalt ragt;
einen das flüssige Material enthaltenden Flüssigkeitsbehälter, von welchem der Kapillarspalt das flüssige Material bezieht.

### Begriffsdefinition:

In der gegenständlichen Patentanmeldung bezieht sich der Begriff "Inhalator" auf medizinische wie nicht-medizinische Inhalatoren. Der Begriff bezieht sich ferner auf Inhalatoren zur Verabreichung von Arzneimitteln und solchen Stoffen, welche nicht als Arzneimittel deklariert sind. Der Begriff bezieht sich außerdem auf Rauchartikel und Zigarettenersatz-Artikel, wie sie beispielsweise in der Europäische Patentklasse A24F47/00B enthalten sind, soweit diese dazu bestimmt sind, dem Benutzer ein Dampf-Luft-Gemisch oder/und Kondensationsaerosol darzureichen. Der Begriff "Inhalator" soll auch keine Einschränkungen dahingehend machen, wie das gebildete Dampf-Luft-Gemisch oder/und Kondensationsaerosol dem Benutzer bzw. dessen Körper zugeführt wird. Das Dampf-Luft-Gemisch oder/und Kondensationsaerosol kann in die Lunge inhaliert werden, oder aber auch nur der Mundhöhle zugeführt werden - ohne Inhalation in die Lunge.

Als "Kapillarspalt" gelte ein jeder Spalt, welcher allein aufgrund der Kapillarwirkung seiner Begrenzungswände einen Flüssigkeitstransport bewirkt. Dochte, ummantelte Dochte oder mit Dochtmaterial befüllte Kanäle sind keine Kapillarspalten.

Die Verwendung des Singulars "Verbund" schließt das Vorhandensein mehrerer Verbunde nicht aus. Die Erfindung schließt Anordungen mit mehreren Verbunden explizit ein.

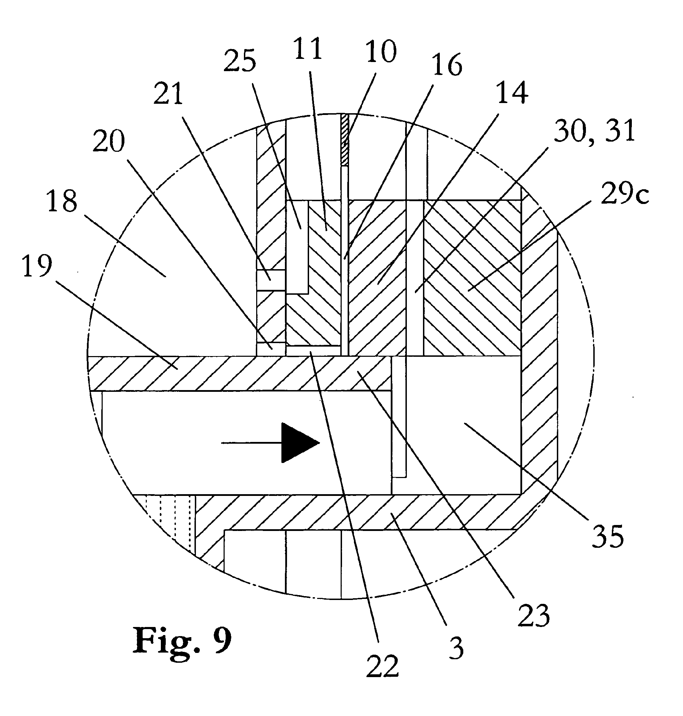

WO 2010/045671 (Helmut Buchberger) beschreibt eine Inhalatorkomponente für die intermittierende, inhalations- oder zugsynchrone Bildung eines Dampf-Luft-Gemisches oder/und Kondensationsaerosols, bestehend aus (Fig. 9-12 und Fig. 17-18) einem Gehäuse 3, eine im Gehäuse 3 angeordnete Kammer 21, eine Lufteinlaßöffnung 26 für die Zufuhr von Luft aus der Umgebung in die Kammer 21, ein elektrisches Heizelement zur Verdampfung einer Portion eines flüssigen Materials 16, wobei der gebildete Dampf sich in der Kammer 21 mit der durch die Lufteinlaßöffnung 26 zugeführten Luft mischt, und sich das Dampf-Luft-Gemisch oder/und Kondensationsaerosol bildet. Die Inhalatorkomponente umfaßt ferner einen Docht mit einer Kapillarstruktur, welcher Docht mit dem Heizelement einen flächigen Verbund 22 bildet und das Heizelement nach einer Verdampfung von neuem selbsttätig mit dem flüssigen Material 16 versorgt. Der flächige Verbund 22 lagert mit zwei Endabschnitten auf zwei elektrisch leitenden, plattenförmigen Kontakten 23, auf deren Oberfläche das Heizelement gleichzeitig elektrisch kontaktiert ist. Die plattenförmigen Kontakte können alternativ auch durch Leiterplatten oder eine gemeinsame Leiterplatte gebildet werden. Zumindest ein beheizter Abschnitt des flächigen Verbundes 22 ist berührungsfrei in der Kammer 21 angeordnet, und die Kapillarstruktur des Dochts liegt im besagten Abschnitt wenigstens auf einer Seite 24 des flächigen Verbundes weitgehend frei. Der flächige Verbund 22 bzw. dessen Docht ragt mit einem Ende in einen Kapillarspalt 41, welcher seinerseits mit einem das flüssige Material 16 enthaltenden Flüssigkeitsbehälter 4 kapillar gekoppelt bzw. koppelbar ist. Der Flüssigkeitsbehälter 4 weist einen offenbaren Verschluß 18 auf, welcher vor Gebrauch noch verschlossen ist. Der öffenbare Verschluß 18 kann von einem Benutzer manuell geöffnet werden, worauf das flüssige Material 16 ein Reservoir 45 flutet und den Kapillarspalt 41 benetzt. Der Kapillarspalt 41 zieht das flüssige Material 16 aus dem Flüssigkeitsbehälter 4 bzw. Reservoir 45 und transportiert es zum Verbund 22. Der Kapillarspalt 41 wird im Grunde durch einen der beiden plattenförmigen Kontakte 23 und ein auf diesen flächig aufgesetztes Oberteil 42 gebildet. Des Weiteren ist in den plattenförmigen Kontakt 23 ein Belüftungskanal 52 eingearbeitet, welcher das Reservoir 45 bzw. den Flüssigkeitsbehälter 4 mit der Kammer 21 verbindet. Der Belüftungskanal 52 bewirkt einen Druckausgleich, indem jede Portion flüssigen Materials 16, welche in den Kapillarspalt 41 gelangt, unmittelbar durch eine volumengleiche Portion Luft ersetzt wird.

Der Flüssigkeitsbehälter 4 ist in der Ansicht nach Fig. 9 oberhalb der den Verbund 22 tragenden, plattenförmigen Kontakte 23 angeordnet. Diese Anordung erweist sich als ausgesprochen raümfordernd und führt dazu, daß die Abmessungen der Inhalatorkomponente relativ groß ausfallen. Ein weiterer Nachteil ist, daß der Kapillarspalt 41 in seiner flächigen Ausdehnung sehr limitiert ist, insofern als bei senkrechter Lage des Kapillarspalts durch das Gewicht der in ihm wirkenden Flüssigkeitssäule im Reservoir 45 ein Unterdruck auftritt, welcher durch die Kapillarität des Belüftungskanals 52 kompensiert werden muß. Reicht die Kapillarität des Belüftungskanals 52 jedoch nicht mehr aus, das Gleichgewicht zu halten, droht das gesamte flüssige Material 16 im Flüssigkeitsbehälter 4 über den Kapillarspalt 41 auszulaufen. Vor allem wenn mehrere Verbunde nebeneinander angeordnet werden sollen (vgl. Fig. 29), oder/und der Docht über zwei voneinander entfernt angeordneten Endabschnitten infiltriert werden soll, ist eine entsprechend große flächige Ausdehnung des Kapillarspalts 41 erforderlich, welche mit der zuvor beschriebenen Anordnung nach WO 2010/045671 aufgrund der aufgezeigten Wirkungen kaum realisierbar ist.

Der Erfindung liegt die Aufgabe zugrunde, die zuvor aufgezeigten Nachteile der aus dem Stand der Technik bekannten Anordnung zu beheben. Der Erfindung liegt insbesondere die Aufgabe zugrunde, eine Inhalatorkomponente der eingangs geschilderten Art so auszugestalten, daß eine vergleichsweise kompakte Gesamtanordnung mit einem entsprechend kleinen Bauvolumen erzielt werden kann. Ferner sollen auch Kapillarspalten mit einer größeren flächigen Ausdehnung vorgesehen werden können.

Die Aufgabe wird durch die kennzeichnenden Merkmale des Patentanspruches 1 gelöst. Demnach ist vorgesehen, daß der Kapillarspalt den Flüssigkeitsbehälter in einer Ansicht senkrecht zur Trägerplatte außen zumindest teilweise überdeckt. Im Sinne der vorliegenden Erfindung gelte auch als "überdeckend", wenn zwischen dem Kapillarspalt und dem Flüssigkeitsbehälter noch weitere Bauteile angeordnet sind. Bedenkt man, daß die den Kapillarspalt bildenden Komponenten senkrecht zur Trägerplatte nur wenig Raum fordern, wird verständlich, daß durch die erfindungsgemäße Anordung Bauraum eingespart werden kann.

In einer Weiterbildung der Erfindung ist vorgesehen, daß der Verbund den Flüssigkeitsbehälter in einer Ansicht senkrecht zur Trägerplatte zumindest teilweise überdeckt. Im Sinne der vorliegenden Erfindung gelte auch als "überdeckend", wenn zwischen dem Verbund und dem Flüssigkeitsbehälter noch weitere Bauteile angeordnet sind. Beachtet man, daß es sich beim Verbund in der Regel um ein relativ dünnes Gebilde handelt, leuchtet ein, daß durch diese weitere Überdeckung nochmals Bauraum eingespart werden kann.

In einer bevorzugten Ausbildung der Erfindung ist vorgesehen, daß die Trägerplatte zumindest abschnittsweise auf dem Flüssigkeitsbehälter lagert. Der Flüssigkeitsbehälter und die Trägerplatte sind also übereinander gestapelt angeordnet. Konstruktiv besonders vorteilhaft ist es, wenn der Flüssigkeitsbehälter im Wesentlichen die Form eines Quaders hat, und die Trägerplatte zumindest abschnittsweise auf einer Seitenfläche des Quaders lagert. Hierdurch kann der zur Verfügung stehende Bauraum in optimaler Weise genutzt werden. Die Trägerplatte besteht vorzugsweise aus einer Leiterplatte, insbesondere aus einer mehrlagigen sogenannten Multilayer-Leiterplatte. Die den elektrischen Heizstrom zu- bzw. abführenden Leiterbahnen können dadurch nämlich auf mehrere Lagen aufgeteilt werden, so daß auch sehr hohe Heizströme weitgehend verlustfrei transportiert werden können.

Die Erfindung betrifft außerdem einen Inhalator, umfassend eine erfindungsgemäße Inhalatorkomponente wie zuvor beschrieben. Die Inhalatorkomponente kann also auch nur ein Teil, insbesondere ein auswechselbares Teil eines Inhalators sein.

Die Erfindung wird anhand eines Ausführungsbeispiels gemäß den Zeichnungen näher erläutert.

Es zeigen:
Fig. 1 einen erfindungsgemäßen Inhalator in verschiedenen Ansichten;
Fig. 2 den Inhalator nach Fig. 1 mit einem wiederverwendbaren Inhalatorteil und einer auswechselbaren Inhalatorkomponente im entkoppelten Zustand;
Fig. 3a und Fig 3b die auswechselbare Inhalatorkomponente in verschiedenen Ansichten;
Fig. 4a, Fig. 4b, Fig. 4c, Fig. 4d und Fig. 4e Schnittansichten der auswechselbaren Inhalatorkomponente längs der Linie A-A in Fig. 3b in verschiedenen Montagezuständen;
Fig. 5 das Detail a aus Fig. 4a in einer vergrößerten Darstellung;
Fig. 6 das Detail b aus Fig. 4b in einer vergrößerten Darstellung;
Fig. 7 eine als Multilayer-Leiterplatte ausgeführte Trägerplatte;
Fig. 8 eine Schnittansicht der auswechselbaren Inhalatorkomponente längs der Linie B-B in Fig. 3b;
Fig. 9 das Detail c aus Fig. 8 in einer vergrößerten Darstellung;
Fig. 10 eine Schnittansicht der auswechselbaren Inhalatorkomponente in Höhe der Verbunde längs der Linie C-C in Fig. 3b.

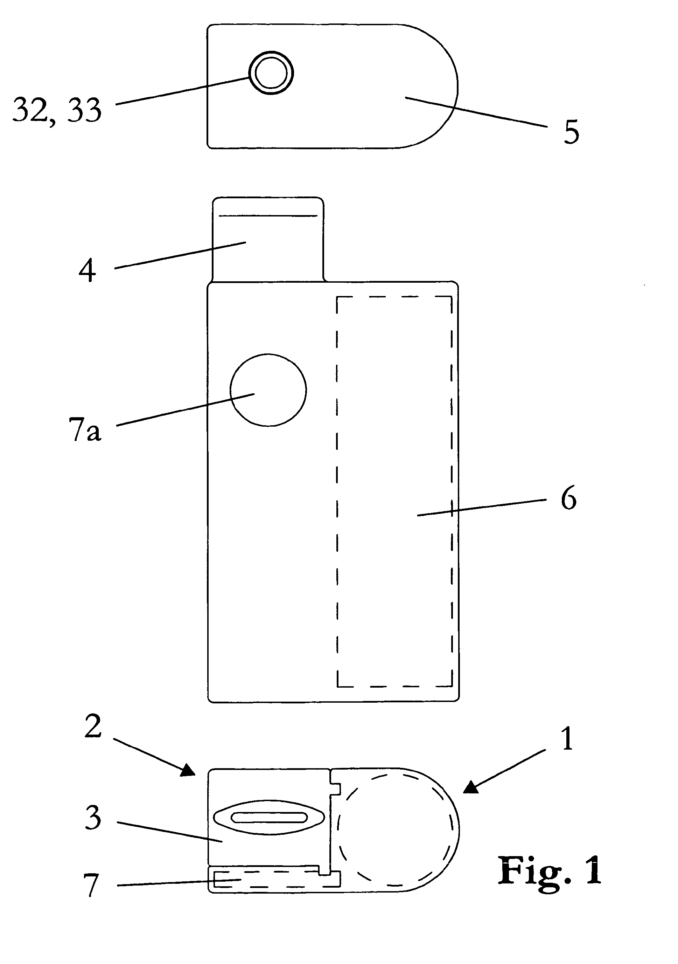

Fig. 1 zeigt einen erfindungsgemäßen Inhalator, dessen Form und Größe derart gestaltet sind, daß der Inhalator von Benutzern einfach und bequem gehandhabt werden kann. Volumenmäßig ist der Inhalator nur etwa halb so groß wie eine Zigarettenschachtel. Der beispielhaft dargestellte Inhalator besteht grundsätzlich aus zwei Teilen, nämlich aus einem Inhalatorteil 1 und einer Inhalatorkomponente 2.

Die Inhalatorkomponente 2 besteht aus einem Gehäuse 3, welches an einer Stirnseite ein tabakpfeifenartiges Mundstück 4 ausbildet. Das Gehäuse 3 ist vorzugsweise aus Kunststoff gefertigt. Die Inhalatorkomponente 2 beinhaltet ein flüssiges Material, welches innerhalb des Gehäuses 3 elektrisch verdampft wird und in ein inhalierbares Dampf-Luft-Gemisch oder/und Kondensationsaerosol übergeführt wird. Das gebildete Dampf-Luft-Gemisch oder/und Kondensationsaerosol wird dem Benutzer über das Mundstück 4 dargeboten. Als flüssiges Material kommen grundsätzlich alle Stoffe und Zubereitungen in Betracht, welche unter atmosphärischen Bedingungen weitgehend rückstandsfrei verdampfen. Diese Bedingung ist auch schon erfüllt, wenn der jeweilige Stoff oder die jeweilige Zubereitung verdünnt, beispielsweise in Wasser oder/ und Ethanol gelöst vorliegt und die Lösung weitgehend rückstandsfrei verdampft. Durch eine hinreichend hohe Verdünnung in einem leicht flüchtigen Lösungsmittel wie Wasser oder/und Ethanol können auch sonst schwer verdampfbare Stoffe die zuvor genannte Bedingung erfüllen, und eine thermische Zersetzung des flüssigen Materials vermieden oder deutlich verringert werden. Die durch Kondensation erzeugten Aerosolteilchen weisen in der Regel einen massemedianen aerodynamischen Durchmesser (MMAD) kleiner als 2µm auf und erreichen dadurch auch die Alveolen. Der erfindungsgemäße Inhalator eignet sich insbesondere für die Verabreichung von systemisch wirkenden Stoffen - insbesondere solchen Wirkstoffen, welche ihre Hauptwirkung im zentralen Nervensystem entfalten. Als Beispiel sei Nikotin erwähnt, dessen Siedepunkt bei 246°C liegt. Die nikotinhaltigen Aerosolpartikel werden vorwiegend in den Bronchien und Alveolen niedergeschlagen, wo der Wirkstoff blitzartig in den Blutkreislauf übergeht. Wenige Sekunden später erreicht das Nikotin in gebündelter Konzentration das Gehirn und kann dort die bekannten Wirkungen entfalten.

Das Inhalatorteil 1 besteht aus einem Hauptgehäuse 5, welches vorzugsweise wieder aus Kunststoff gefertigt ist. Das Hauptgehäuse 5 beinhaltet zumindest eine Batterie 6 und einen elektrischen Schaltkreis 7 (in Fig. 1 strichliert dargestellt) samt Schalter 7a. Die Batterie 6 und der elektrische Schaltkreis 7 stellen die für die Verdampfung des flüssigen Materials erforderliche elektrische Energie bereit. Die Batterie 6 besteht vorzugsweise aus einem wiederaufladbaren Akkumulator, beispielsweise vom Typ CGR18650K des Herstellers Panasonic, www.industrial.panasonic.com. Hierbei handelt es sich um eine zylindrische Lithium-Ionen-Zelle der Baugröße 18650 mit einer Speicherkapazität von 1650mAh und einer Strom-Belastbarkeit von bis zu 30A. Vergleichbare Zellen werden auch von anderen Herstellern, u.a. Sony, Samsung, LG Chem, in großen Stückzahlen gefertigt.

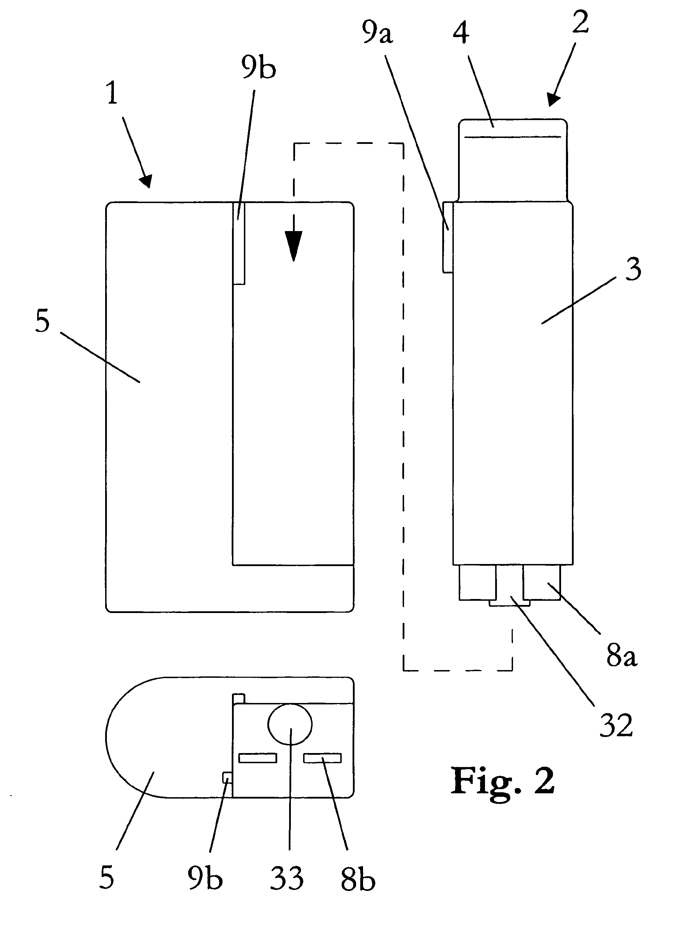

Wie die Fig. 2 zeigt, sind das Inhalatorteil 1 und die Inhalatorkomponente 2 im konkreten Ausführungsbeispiel voneinander lösbar ausgeführt. Diese Anordnung macht das Inhalatorteil 1 wiederverwendbar, was grundsätzlich sinnvoll ist, wenn man in Betracht zieht, daß erstens das Inhalatorteil 1 mit dem flüssigen Material nicht in Berührung kommt, also nicht mit dem flüssigen Material kontaminiert wird, und zweitens Komponenten beinhaltet, welche langlebiger sind als die Bestandteile der Inhalatorkomponente 2. Die Inhalatorkomponente 2 wird, nachdem das flüssige Material aufgebraucht ist, vom Benutzer als Ganzes sachgerecht entsorgt, und durch eine neue Inhalatorkomponente 2 ersetzt. Die Inhalatorkomponente 2 stellt insofern einen auswechselbaren Einwegartikel dar. Eine sachgerechte Entsorgung ist vor allem dann angezeigt, wenn das flüssige Material Arzneimittel oder Gifte wie Nikotin enthält. Grundsätzlich wäre es natürlich auch denkbar, das Inhalatorteil 1 und die Inhalatorkomponente 2 einteilig, also voneinander untrennbar auszuführen. Diese Ausführungsform dürfte jedoch unwirtschaftlicher sein, weil in diesem Fall alle Teile und Komponenten des Inhalators, also der Inhalator als Ganzes einen Einwegartikel zur einmaligen Benutzung bildet. Selbstverständlich schließt die gegenständliche Erfindung auch diese Ausführungsform mit ein, wobei in diesem Fall der ganze Inhalator als Inhalatorkomponente aufzufassen ist.

Die mechanische Kopplung zwischen der auswechselbaren Inhalatorkomponente 2 und dem wiederverwendbaren Inhalatorteil 1 erfolgt über Steckzungen 8a und durch das Gehäuse 3 gebildete Führungsnasen 9a, welche in korrespondierende, durch das Hauptgehäuse 5 des wiederverwendbaren Inhalatorteils 1 gebildete Steckbuchsen 8b und Führungsnuten 9b eingreifen. Die Steckzungen 8a und Steckbuchsen 8b dienen gleichzeitig zur Einleitung der elektrischen Energie in die auswechselbare Inhalatorkomponente 2 zur Verdampfung des flüssigen Materials, wie nachfolgend noch detaillierter gezeigt wird.

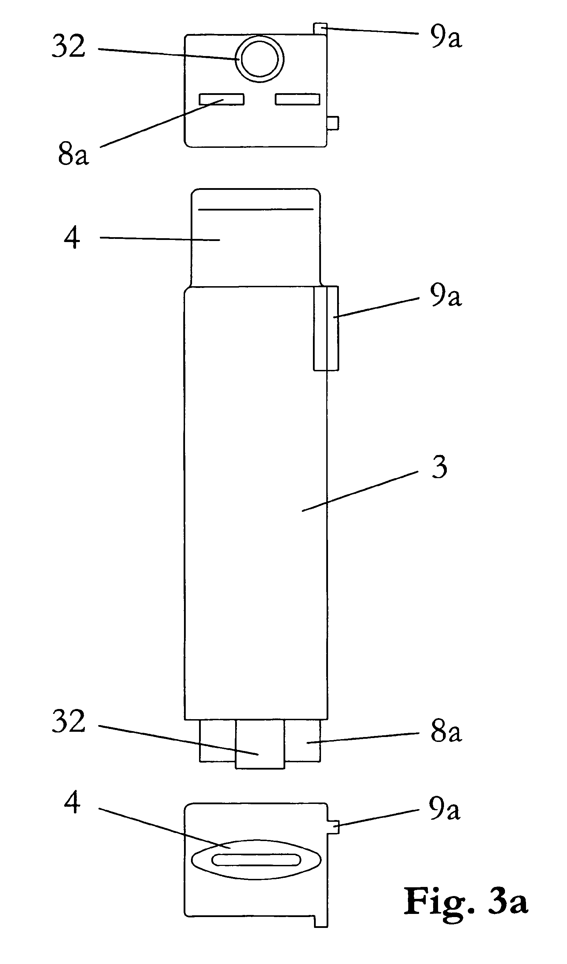

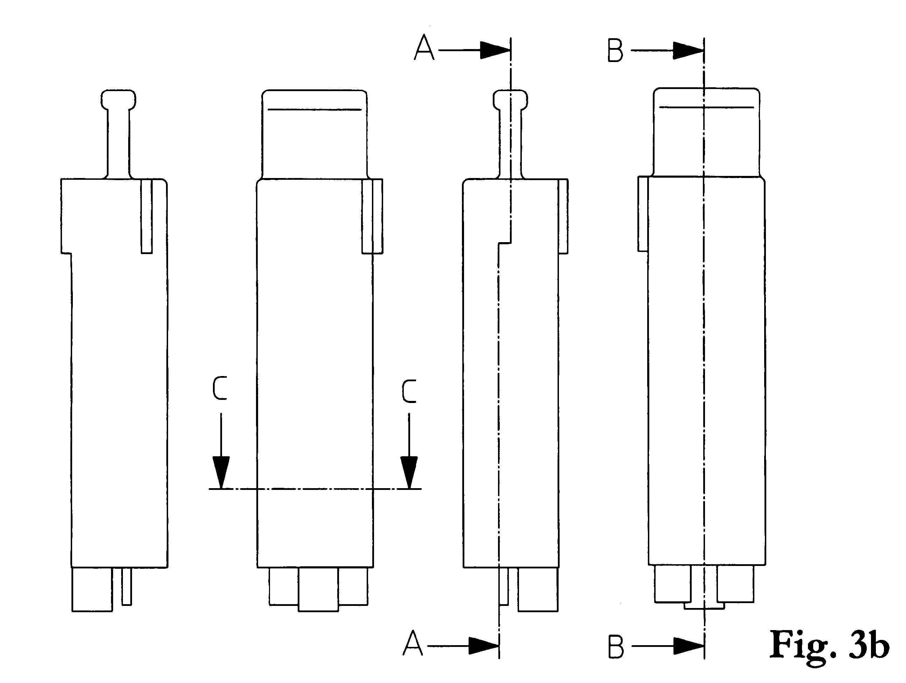

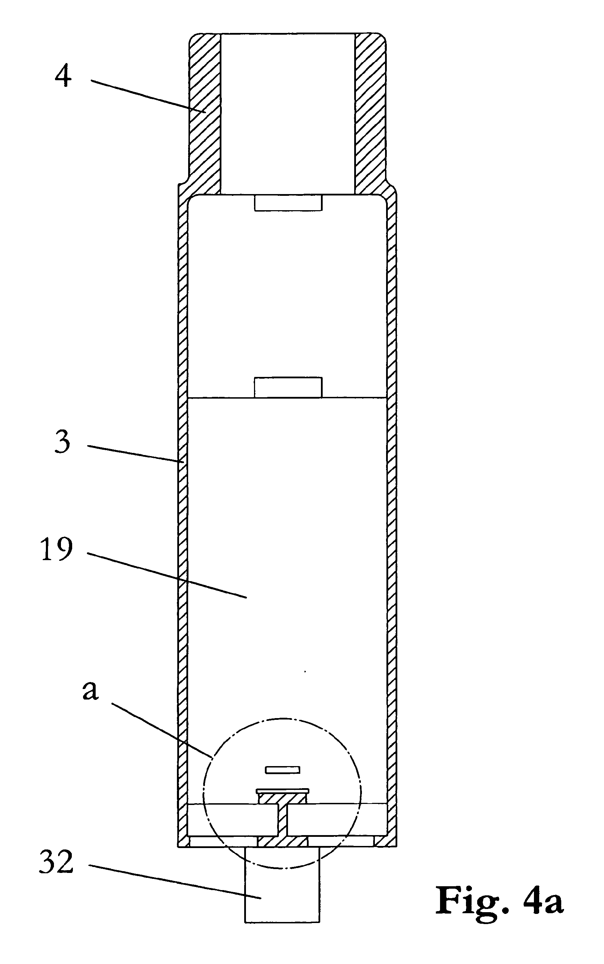

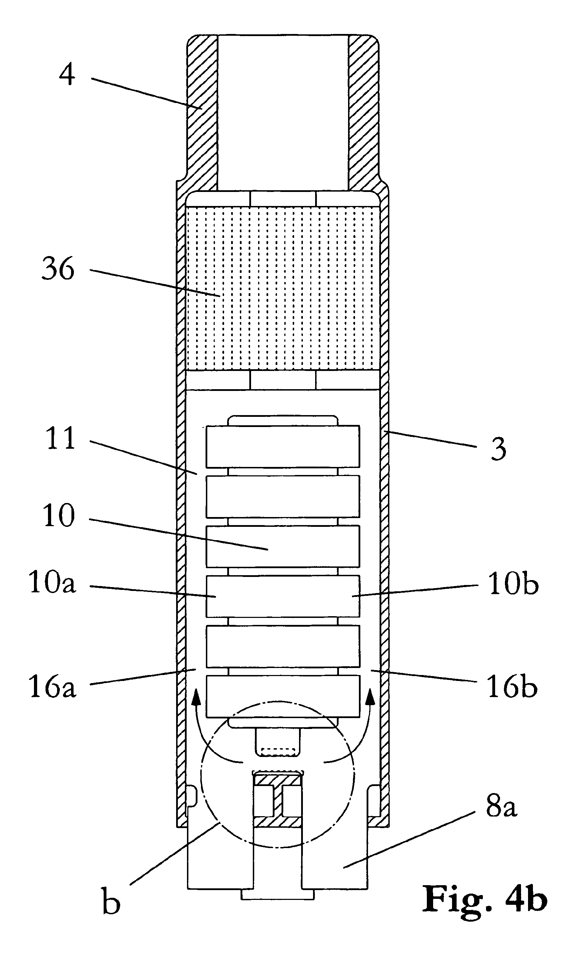

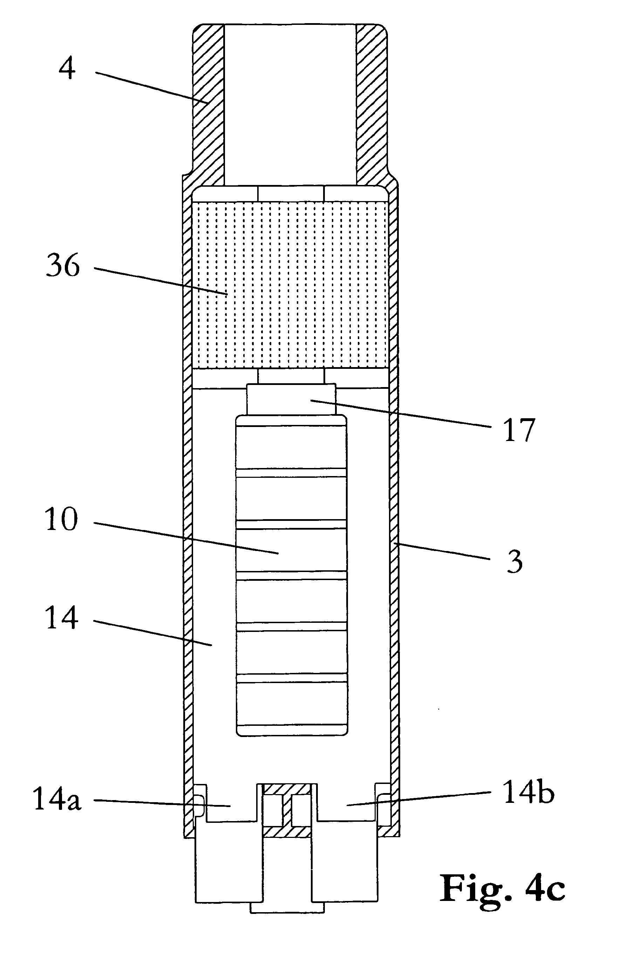

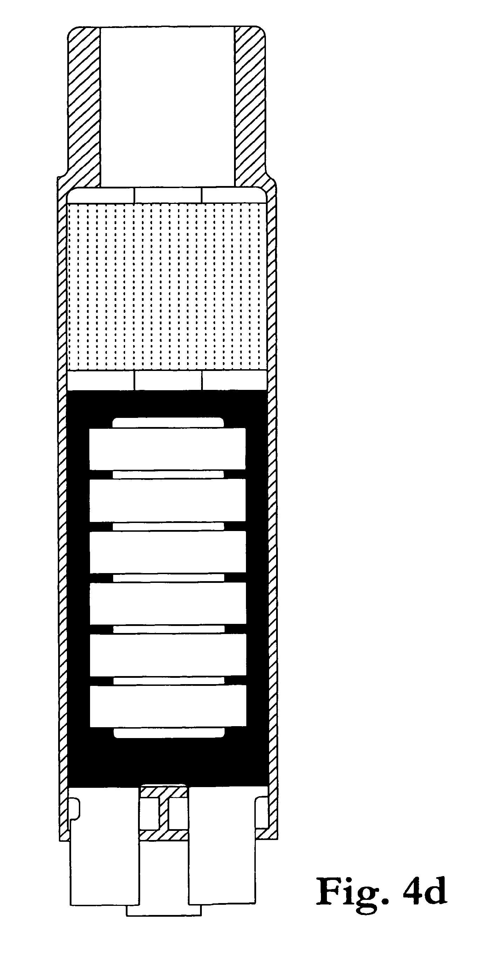

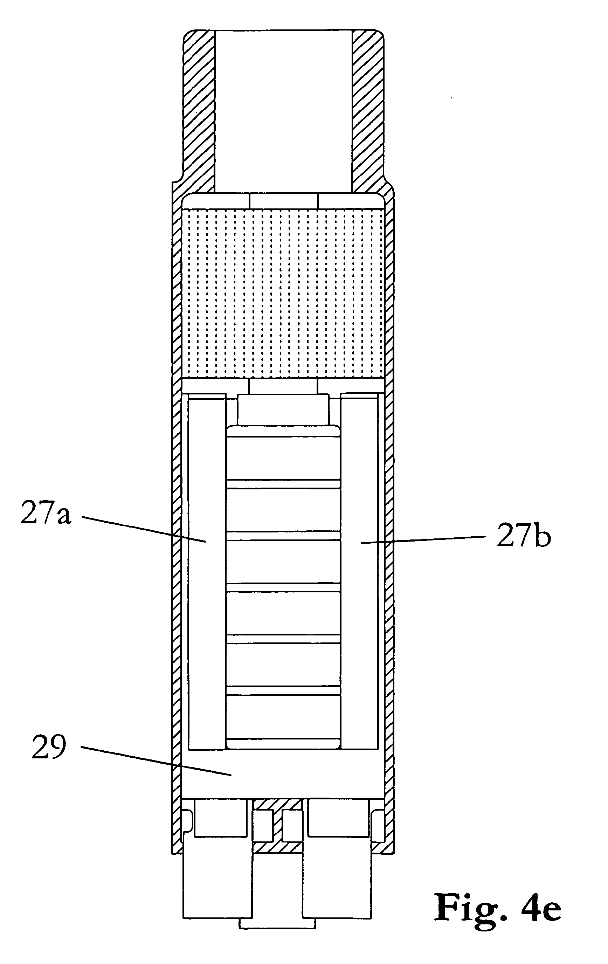

Fig. 3a und Fig. 3b zeigen verschiedene Ansichten der auswechselbaren Inhalatorkomponente 2. Die Fig. 4-9 geben weiteren Aufschluss über den inneren Aufbau der Inhalatorkomponente 2. Demnach weist das Gehäuse 3 der Inhalatorkomponente 2 im Wesentlichen eine quaderförmige Gestalt auf. Im Inneren des quaderförmigen Gehäuses 3 befinden sich die für die Bildung des Dampf-Luft-Gemisches oder/und Kondensationsaerosols wesentlichen Komponenten. Zu diesen zählen insbesondere die Verbunde 10, welche die Verdampfung des flüssigen Materials bewirken. Im konkreten Ausführungsbeispiel sind sechs Verbunde 10 nebeneinander angeordnet, und die Verbunde haben eine flächige Gestalt. Die flächigen Verbunde 10 bestehen jeweils aus einem Docht und einem elektrischen Heizelement, welche miteinander flächig verbunden oder flächig ineinander integriert sind. Die flächigen Verbunde 10 können beispielsweise durch eine Metallfolie und darauf aufgesinterte Metallgewebelagen gebildet werden. Statt des Metallgewebes können auch offenporige Metallschäume Verwendung finden. Die offenporige Kapillarstruktur der auf die Metallfolie aufgesinterten Gewebelagen bzw. des Metallschaums bildet den Docht, und der elektrische Widerstand des Metalls bildet das Heizelement. Geeignete metallische Widerstandsmaterialien sind beispielsweise Edelstähle wie AISI 304 oder AISI 316 sowie Heizleiterlegierungen, insbesondere NiCr-Legierungen. Die Herstellung solcher flächigen Verbunde 10 zählt zum Stand der Technik und wird beispielsweise in der bereits zitierten WO 2010/045671 (Helmut Buchberger) im Detail offenbart.

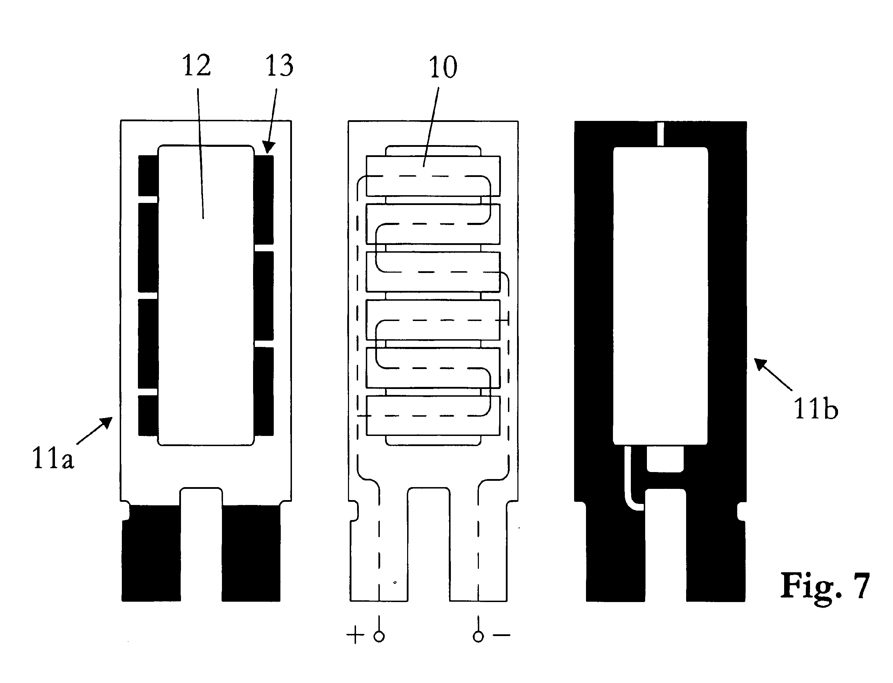

Wie Fig. 4b und Fig. 7 am besten zeigen, lagern die flächigen Verbunde 10 mit zwei Endabschnitten 10a, 10b auf einer Trägerplatte 11. Die Trägerplatte 11 weist eine große Ausnehmung 12 auf, welche von den Verbunden 10 berührungsfrei überspannt wird. Die Trägerplatte 11 ist im konkreten Ausführungsbeispiel als Leiterplatte, insbesondere als Multilayer-Leiterplatte ausgeführt. Als Material für die Leiterplatte 11 eignen sich grundsätzlich alle bekannten Leiterplatten-Werkstoffe, insbesondere die Werkstofftypen FR1 bis FR5. Die flächigen Verbunde 10 sind im Bereich der Endabschnitte 10a, 10b auf Leiterbahnen 13 der Leiterplatte 11 elektrisch kontaktiert. In Fig. 7 sind die Leiterbahnen 13 als schwarze Flächen dargestellt. Im Fall der zuvor beschriebenen Metallfolien-Verbunde erfolgt die elektrische Kontaktierung vorzugsweise durch eine folienseitige Lötung, gegebenenfalls nach Vorbehandlung mit einem geeigneten Flussmittel. Edelstähle der Werkstoff-Qualitäten AISI 304 und AISI 316 können beispielsweise mit einem Lötkonzentrat mit der Handelbezeichnung "5050S-Nirosta" der Firma Stannol GmbH, www.stannol.de, problemlos gelötet werden. Die elektrische Kontaktierung kann alternativ aus einer Klebeverbindung mittels eines elektrisch leitenden Klebstoffes, zum Beispiel mittels eines silberhaltigen Klebers auf Epoxidbasis, bestehen. Die Bestückung der Leiterplatte 11 mit den flächigen Verbunden 10 sowie deren Kontaktierung erfolgen vollautomatisch, wobei Verfahren der Leiterplattenindustrie angewandt werden können, welche Verfahren sich im Übrigen auch für eine Massenfertigung eignen.

Die Leiterplatte 11 ragt aus dem Gehäuse 3 in Form der bereits früher erwähnten Steckzungen 8a heraus. Die beiden Steckzungen 8a dienen zur Einleitung der elektrischen Energie in die Inhalatorkomponente 2. Die elektrische Energie wird den Verbunden 10 über die Leiterbahnen 13 zugeführt. Nach Fig. 7 sind die Leiterbahnen 13 sowohl auf der Vorderseite 11a als auch auf der Rückseite 11b der Leiterplatte 11 angeordnet, wobei die Vorderseite 11a die Bestückungsseite ist - das ist jene Seite, auf welcher die Verbunde 10 kontaktiert sind. Weitere Leiterbahnen können optional auch noch in Zwischenlagen angeordnet werden. Die einzelnen Leiterbahn-Lagen sind nach dem Stand der Technik mittels sogenannter Durchkontaktierungen zweckmäßig miteinander verbunden. In Fig. 7 ist ferner der Stromfluß dargestellt. Demnach sind im konkreten Beispiel jeweils drei Verbunde 10 miteinander in Reihe geschaltet. Dadurch kann auf den resultierenden Heizwiderstand und damit auf die Heizleistung und Verdampfungrate in gewissen Grenzen Einfluß genommen werden. Es kann auch vorgesehen sein, daß die elektrischen Einzelwiderstände der sechs Verbunde 10 unterschiedlich groß sind, indem beispielsweise die Dicke der Metallfolie entsprechend variiert wird. Durch diese Maßnahme kann der Verdampfungsprozeß ähnlich wie in einer Zigarette auch vom Ort abhängig gemacht werden.

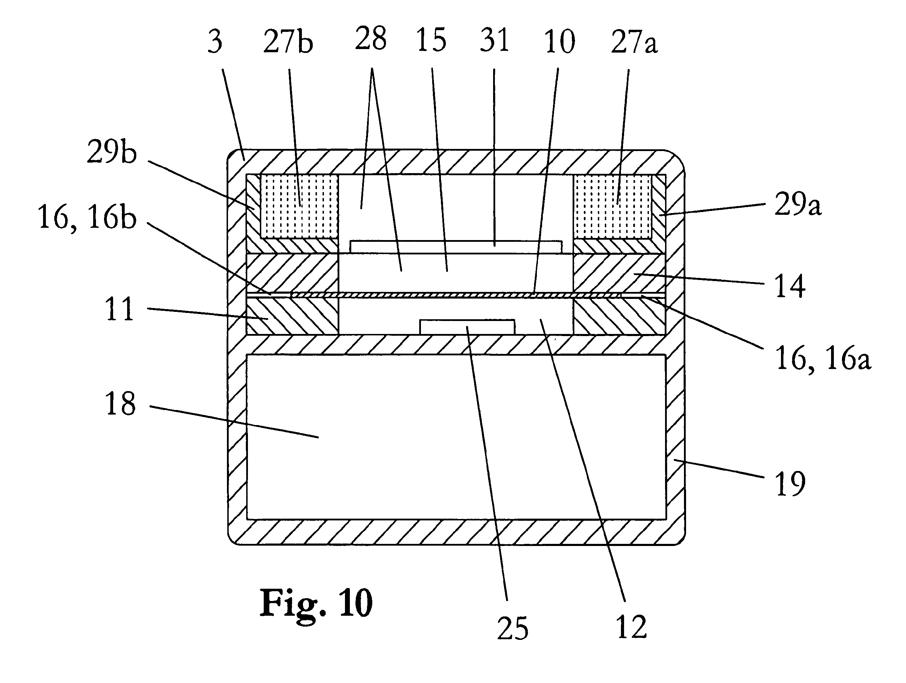

Auf die Vorderseite 11a der Leiterplatte 11 ist ein im Wesentlichen plattenförmiges, vorzugsweise aus Kunststoff bestehendes Oberteil 14 aufgesetzt (siehe Fig. 4c und Fig. 8-10). Das Oberteil 14 weist eine Aussparung 15 auf, welche hinsichtlich ihrer Größe und Anordnung mit der Ausnehmung 12 in der Leiterplatte 11 korreliert. Im einfachsten Fall lagert das Oberteil 14 direkt auf den Endabschnitten 10a, 10b der flächigen Verbunde 10. Hierdurch bildet das Oberteil 14 zusammen mit der Leiterplatte 11 einen Kapillarspalt 16, dessen lichte Weite bzw. Spaltbreite im Wesentlichen der Dicke der flächigen Verbunde 10 entspricht (siehe Fig. 9 und Fig. 10). Die Spaltbreite beträgt typischerweise 0,2mm. In Fig. 4d ist die flächige Ausdehnung des Kapillarspalts 16 als schwarze Fläche dargestellt. Das Oberteil 14 ist auf der Leiterplatte 11 durch eine Klebeverbindung befestigt, und zwar über zwei Vorsprünge 14a, 14b und über einen Haltewinkel 17.

Die Leiterplatte 11 lagert mit ihrer Rückseite 11b auf einem das flüssige Material 18 enthaltenden Flüssigkeitsbehälter 19 (siehe Fig. 4a/4b, Fig. 8 und Fig. 10). Der Flüssigkeitsbehälter 19 bzw. dessen Wand wird durch das Gehäuse 3 gebildet und weist eine quaderförmige Gestalt auf. Die Leiterplatte 11 ist vorzugsweise mittels einer Klebeverbindung auf der Flüssigkeitsbehälterwand befestigt. Die Befüllung des Flüssigkeitsbehälters 19 mit dem flüssigen Material 18 erfolgt werkseitig am Ende des Fertigungsprozesses vorzugsweise über ein kleines Loch in der Behälterwand (nicht dargestellt) vollautomatisch mittels einer Kanüle und einer Dosiereinheit. Das Loch wird nach der Befüllung verschlossen, zum Beispiel zugeschmolzen, und die ganze Inhalatorkomponente 2 luftdicht verpackt.

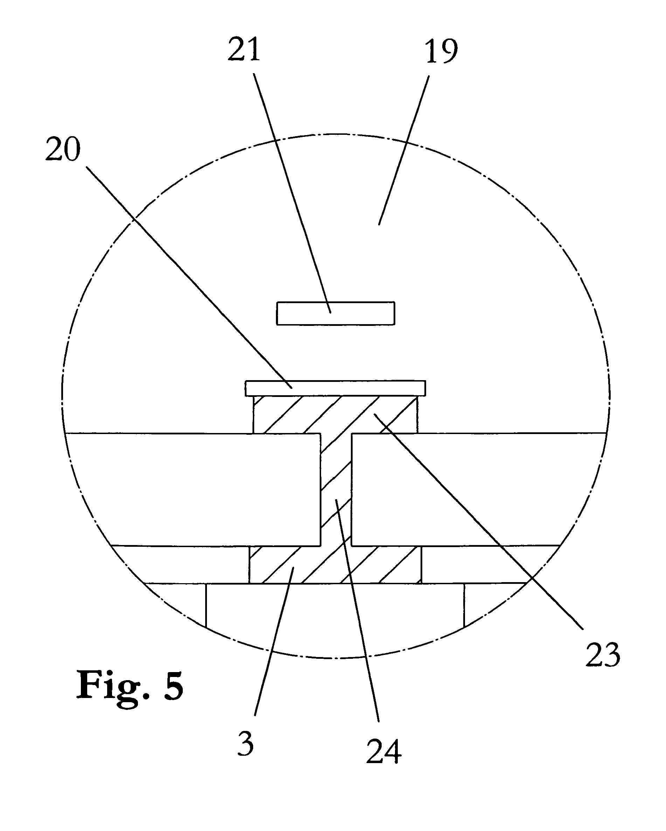

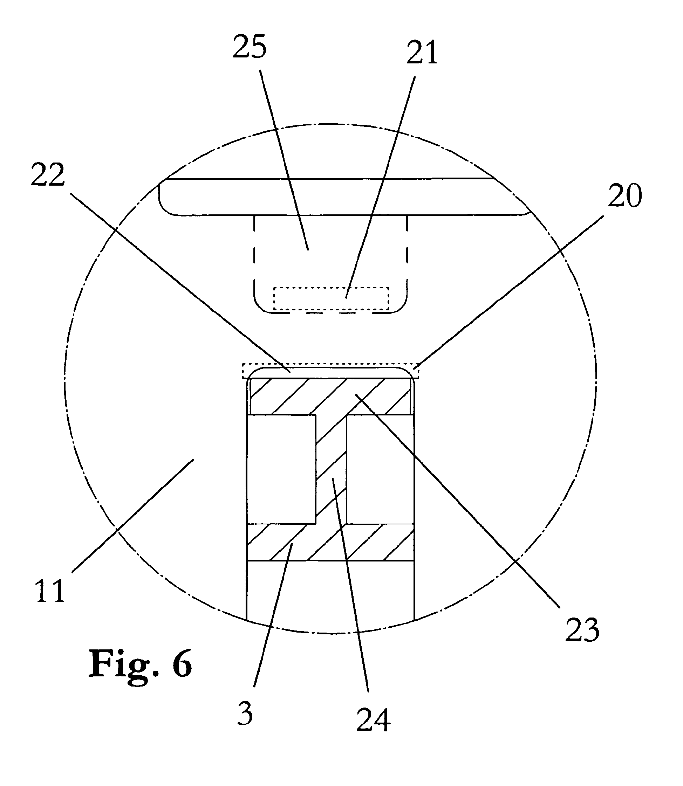

Der Flüssigkeitsbehälter 19 weist an seinem unteren Ende zwei eng nebeneinander angeordnete Öffnungen auf - die Versorgungsöffnung 20 und die Belüftungsöffnung 21 (siehe Fig. 5, Fig. 6 und Fig. 9). Die Versorgungsöffnung 20 korrespondiert mit einer Durchtrittsöffnung 22, welche durch den Rand der Leiterplatte 11 und einen Fortsatz 23 der Flüssigkeitsbehälterwand gebildet wird (siehe Fig. 6 und Fig. 9). Der Fortsatz 23 bildet gleichzeitig einen Anschlag für das Oberteil 14. Zur Aussteifung stützt sich der Fortsatz 23 über einen Steg 24 am Gehäuse 3 ab. Die Versorgung des Kapillarspalts 16 mit dem flüssigen Material 18 erfolgt über die Versorgungsöffnung 20 und die Durchtrittsöffnung 22 und wird durch die im Kapillarspalt 16 wirkenden Kapillarkräfte angetrieben. Damit diese Kapillarkräfte überhaupt wirken können, ist es erforderlich, daß das flüssige Material 18 sämtliche beaufschlagten Oberflächen gut benetzt. Um dies sicherzustellen, sind die betroffenen Bauteile - das sind der Flüssigkeitsbehälter 19, die Leiterplatte 11 samt Verbunden 10 und das Oberteil 14 - noch vor der Montage in einem geeigneten Prozeß zu hydrophilieren. Geeignete Prozesse sind das Hydrophilieren im Sauerstoff-Plasma sowie das Hydrophilieren mittels Plasmapolymerisation. Beide Prozesse werden beispielsweise von der Firma Diener electronic GmbH u. Co. KG, www.plasma.de, im Rahmen von Lohnaufträgen angeboten. Die genannte Firma ist außerdem in der Lage, entsprechende, auch für eine Massenfertigung taugliche Anlagen kundenspezifisch zu planen und zu errichten.

Die Belüftungsöffnung 21 korrespondiert mit einer in die Leiterplatte 11 eingebrachten Belüftungsnut 25, welche ihrerseits über die Ausnehmung 12 mit einem unter Atmosphärendruck stehenden Innenraum kommuniziert. Die Belüftungsöffnung 21 und die Belüftungsnut 25 bewirken einen Druckausgleich, indem jede Portion flüssigen Materials 18, welche in den Kapillarspalt 16 gelangt, unmittelbar durch eine volumengleiche Portion Luft ersetzt wird.

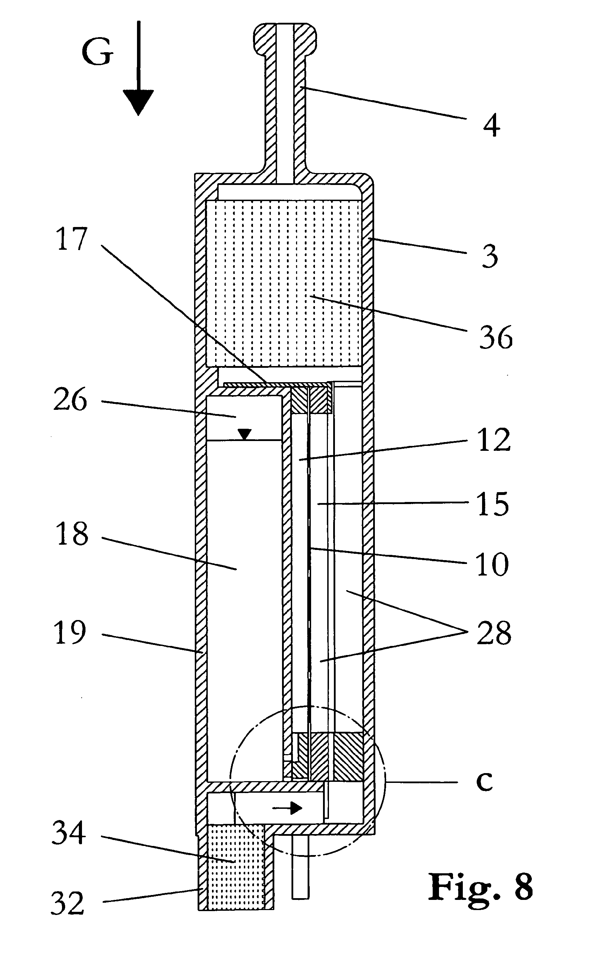

Die überlappende Anordnung der Leiterplatte 11 und des Flüssigkeitsbehälters 19, sowie die zuvor beschriebene Anordnung der Versorgungsöffnung 20, der Durchtrittsöffnung 22 und der Belüftungsöffnung 21 gestatten es, eine vergleichsweise große Kapillarspaltfläche vorzusehen, welche erforderlich ist, wenn mehrere nebeneinander angeordnete Verbunde 10 mit dem flüssigen Material 18 zu versorgen sind. Die Gefahr, daß an irgendeiner Stelle durch das Wirken der Gravitation flüssiges Material 18 austritt, kann weitestgehend abgewendet werden. In der in Fig. 8 dargestellten senkrechten Lage der Inhalatorkomponente 2 (ein Pfeil zeigt die Wirkrichtung der Gravitation an) herrscht in der Belüftungsöffnung 21 ännähernd Atmosphärendruck, da sich der Kapillarspalt 16 in Bezug auf die Durchtrittsöffnung 22 nach unten hin nicht weiter ausdehnt (vgl. Fig. 4d). In einer auf den Kopf gestellten Lage der Inhalatorkomponente 2 (das Mundstück 4 weist nach unten) kann die Flüssigkeitssäule im Kapillarspalt 16 zwar einen Unterdruck induzieren, dieser kann jedoch auf das flüssige Material 18 im Flüssigkeitsbehälter 19 nicht zurückwirken, weil ein Luftpolster im Flüssigkeitsbehälter 19 die kapillare Kopplung unterbricht. Bei der werkseitigen Befüllung des Flüssigkeitsbehälters 19 ist lediglich darauf zu achten, daß ein kleines Luftvolumen 26 zur Bildung des Luftpolsters im Behälter verbleibt.

Bevor auf die Funktionsweise des erfindungsgemäßen Inhalators näher eingegangen wird, sollen im Folgenden noch weitere Bestandteile der Inhalatorkomponente 2 beschrieben werden. Auch wenn diese Bestandteile nicht unmittelbar erfindungsrelevant sein mögen, so trägt die Beschreibung derselben doch dazu bei, die Funktion der erfindungsgemäßen Inhalatorkomponente im Ganzen noch besser zu verstehen, und die Ausführbarkeit der Erfindung noch sicherer zu gewährleisten: zwischen dem Oberteil 14 und dem Gehäuse 3 sind zwei offenporige, saugfähige Schwämme 27a, 27b (siehe Fig. 4e und Fig. 10) angeordnet. Der Raum zwischen den Schwämmen bildet zusammen mit der Aussparung 15 eine Kammer 28 (vgl. auch Fig. 8), in welcher die eigentliche Bildung des Dampf-Luft-Gemisches oder/und Kondensationsaerosols stattfindet. Die Schwämme 27a, 27b nehmen in ihren Poren aus der Dampfphase gebildete Kondensatablagerungen auf und verhindern, daß sich in der Inhalatorkomponente 2 frei bewegliche Kondensatansammlungen bilden, welche die Funktion der Inhalatorkomponente beeinträchtigen könnten. Solche Kondensatansammlungen können auch aus hygienischer Sicht ein Problem darstellen, insbesondere wenn sie über das Mundstück 4 in die Mundhöhle eines Benutzers gelangen. Die Schwämme 27a, 27b bestehen vorzugsweise aus einem feinporigen Faserverbund. Die Firma Filtrona Fibertec GmbH, www.filtronafibertec.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet weden.

Die Schwämme 27a, 27b lagern auf von einem U-förmigen Träger 29 gebildeten Winkelprofilen 29a, 29b (siehe Fig. 4e und Fig. 10). Der Träger 29 ist mit dem Oberteil 14 durch eine Klebeverbindung gefügt. Der Träger 29 samt Winkelprofilen 29a, 29b besteht vorzugsweise aus einem hydrophoben Kunststoff. Das hydrophobe Material wirkt wie eine Flüssigkeitssperre und stellt sicher, daß kein flüssiges Material 18 durch Kapillareffekte zu den Schwämmen 27a, 27b gelangen kann. In den die Winkelprofile 29a, 29b verbindenden Schenkel 29c ist auf der dem Oberteil 14 zugewandten Seite eine Vertiefung 30 eingearbeitet, welche zusammen mit dem Oberteil 14 eine Luftdüse 31 bildet (siehe Fig. 9 und Fig. 10). Die Luftdüse 31 dient, wie später noch näher dargestellt wird, der Einbringung von Umgebungsluft in die Kammer 28. Damit Kondensatablagerungen die Luftdüse 31 nicht blockieren, empfiehlt es sich, die Oberfäche des Oberteils 14 im Bereich der Luftdüse 31 mit einem dünnen hydrophoben Klebeband zu überkleben (nicht dargestellt).

Die Versorgung der Inhalatorkomponente 2 mit Umgebungsluft zur Bildung des Dampf-Luft-Gemisches oder/und Kondensationsaerosols erfolgt über einen durch das Gehäuse 3 gebildeten Ansaugschnorchel 32 (siehe Fig. 3a/3b und Fig.8). Der Ansaugschnorchel 32 ist auf der dem Mundstück 4 gegenüberliegenden Seite der Inhalatorkomponente 2 angeordnet. Diese Lage schützt am ehesten vor dem Eintritt von Regenwasser. Im gekoppelten Zustand ragt der Ansaugschnorchel 32 der Inhalatorkomponente 2 durch ein durch das Hauptgehäuse 5 des Inhalatorteils 1 gebildetes Loch 33 (siehe Fig. 2). Im Ansaugschnorchel 32 befindet sich eine Strömungsdrossel 34. Die Strömungsdrossel 34 hat den Zweck, einen Strömungswiderstand zu erzeugen, welcher dem einer Zigarette ähnlich ist, so daß der Benutzer während eines Zuges einen ähnlichen Zugwiderstand verspürt wie bei einem Zug an einer Zigarette. Konkret sollte der Strömungswiderstand bei einem Durchfluß von 1,05 L/min im Bereich 8-16mbar liegen und eine möglichst lineare Charakteristik aufweisen. Die Strömungsdrossel 34 ist erforderlich, wenn das gebildete Dampf-Luft-Gemisch oder/und Kondensationsaerosol wie bei einer Zigarette zugeführt werden soll, nämlich als Zug in die Mundhöhle (Zugvolumen: ca. 20-80mL), gegebenenfalls gefolgt von einer Inhalation in die Lunge. Diese Betriebsweise empfiehlt sich vor allem dann, wenn das flüssige Material 18 Nikotin enthält. Die Strömungsdrossel 34 entfällt jedoch, wenn der Inhalator eine direkte Lungeninhalation in einem einzigen Schritt gestatten soll, wie dies bei den meisten medizinischen Inhalatoren der Fall ist. Die Strömungsdrossel 34 besteht vorzugsweise aus einem zigarettenfilterähnlichen Faserverbund, wobei die Dichte des Materials auf die zuvor genannte Durchflußcharakteristik abzustimmen ist. Das Material kann wiederum von der Firma Filtrona Fibertec GmbH, www.filtronafibertec.com, bezogen werden.

Im Folgenden soll die Funktion des Inhalators im Detail beschrieben werden: ein Benutzer koppelt eine neue Inhalatorkomponente 2 mit dem wiederverwendbaren Inhalatorteil 1. Der elektrische Schaltkreis 7 registriert die Kopplung und veranlaßt gegebenenfalls die Ausführung bestimmter vorbereitender Operationen, beispielsweise eines oder mehrerer Verdampfungszyklen mit dem Ziel, die Verbunde 10 mit frischem flüssigen Material 18 zu versorgen oder/und stationäre Bedingungen herzustellen. Sobald diese Operationen abgeschlossen sind, signalisiert der elektrische Schaltkreis 7 zum Beispiel über eine Leuchtdiode die Betriebsbereitschaft des Inhalators. Der Benutzer führt das Mundstück 4 des Inhalators an den Mund und betätigt den Schalter 7a. Gleichzeitig beginnt er am Mundstück 4 zu ziehen. Der hierdurch erzeugte Unterdruck bewirkt, daß Luft aus der Umgebung in den Ansaugschnorchel 32 strömt. Nachdem die Luft die Strömungsdrossel 34 durchsetzt hat, biegt die Strömung im rechten Winkel ab (siehe Pfeile in Fig. 8 und Fig. 9) und mündet in eine Plenumkammer 35, wo sich die Luft sammelt und sodann gleichmäßig der schlitzförmigen Luftdüse 31 zugeführt wird. Die Luftströmung wird in der Luftdüse 31 beschleunigt und tritt mit einer hohen Mündungsgeschwindigkeit in die Kammer 28 ein.

Die Betätigung des Schalters 7a bewirkt, daß der Schaltkreis 7 den Heizstrom einschaltet. Der Heizstrom wird vorzugsweise mittels Leistungs-MOSFET geschaltet, wobei die zugeführte Leistung durch eine Taktung (Duty Cycle) den jeweiligen Erfordernissen angepaßt werden kann. Diese Anpassung kann in bestimmten Grenzen auch durch den Benutzer über eine Schnittstelle erfolgen, wodurch es diesem ermöglicht wird, auf die erzeugte Aerosol- bzw. Rauchmenge Einfluß zu nehmen. Der Heizstrom wird für eine voreingestellte Zeitperiode ("Heizperiode") geschaltet, welche typischerweise 1,0-1,8 Sekunden beträgt. Der Heizstrom wird den Verbunden 10 über die Steckzungen 8a und die Leiterbahnen 13 der Leiterplatte 11 zugeführt und bewirkt eine blitzartige Aufheizung der Verbunde 10 und des in den Dochten gespeicherten flüssigen Materials 18, woraufhin das flüssige Material 18 verdampft. Der Dampf wird in die Kammer 28 emittiert, wo er sich mit der durch die Luftdüse 31 einströmenden Luft mischt. Die Anordnung und Dimensionierung der Luftdüse 31 bewirkt eine gleichmäßige und schnelle Überströmung der Verbunde 10. Hierdurch wird sichergestellt, daß der von den Verbunden 10 freigesetzte Dampf allseits annähernd gleiche Mischungsbedingungen vorfindet, und die Mischung von Dampf und Luft innig ist. Die Luft bewirkt eine Kühlung des Dampfes, so daß sich außerdem ein Kondensationsaerosol ausbilden kann, sofern das verdampfte flüssige Material 18 Substanzen mit hinreichend niedrigem Dampfdruck - sogenannte aerosolbildende Substanzen - enthält. Ein typisches Beispiel für solche aerosolbildenden Substanzen ist Glycerol.

Das in der Kammer 28 gebildete Dampf-Luft-Gemisch oder/und Kondensationsaerosol durchströmt schließlich im Ausführungsbeispiel noch einen Kühler 36, bevor es dem Benutzer über das Mundstück 4 zur Inhalation dargeboten wird (siehe Fig. 4e und Fig. 8). Der Kühler 36 kann beispielsweise aus einem porösen Füllmaterial, einem vliesartigen Fasermaterial oder aus einem offenzelligen Schaummaterial bestehen, dessen Poren vom gebildeten Dampf-Luft-Gemisch oder/und Kondensationsaerosol durchströmt werden. Der Kühler 36 kann auch mehrstufig ausgeführt sein, wobei die einzelnen Kühlerstufen unterschiedliche Eigenschaften aufweisen. Enthält das zu verdampfende Material Nikotin, kann es von Vorteil sein, das Kühlermaterial zumindest einer Kühlerstufe mit einem geeigneten Absorbens, beispielsweise mit Zitronensäure zu beschichten. Das Absorbens entzieht dem durchströmenden Kondensationsaerosol leichtflüchtige Nikotinfraktionen, welche ansonsten in der Mundhöhle und im Rachen niedergeschlagen würden, was weder pharmakokinetisch noch organoleptisch wünschenswert ist. Dem Kühlermaterial können ferner Aromastoffe wie Menthol zugefügt werden.

Geeignete vliesartige Fasermaterialien können beispielsweise von der Firma Freudenberg Vliesstoffe KG, www.freudenberg-filter.com, bezogen werden. Das unter der Bezeichnung Viledon®-Filtermatten vertriebene und aus Polyolefinfasern bestehende Material wird nach Kundenspezifikation angefertigt, wobei die Materialeigenschaften so abgestimmt werden können, daß das Endprodukt für die feinen Partikel des erzeugten Kondensationsaerosols weitestgehend durchlässig ist. Ein geeignetes Schaummaterial kann zum Beispiel von der Firma Dunlop Equipment, www.dunlop-equipment.com bezogen werden. Der angeführte Lieferant bietet Ni- und NiCr-Schaum unter der Produktbezeichnung Retimet® (Grade 80) mit einer Porosität von 90-95% und einem Porendurchmesser von etwa 300µm in Plattenform bis zu Dicken von 15mm an. Nach mündlicher Mitteilung von Firmenvertretern können aus technologischer Sicht auch noch etwas feinporigere Schäume hergestellt werden. Die Metallschäume können außerdem durch Walzen zusätzlich verdichtet werden. Die Platten können durch Laserschneiden oder Drahterodieren weiterverarbeitet werden. Ni-Schaum und insbesondere NiCr-Schaum zeichnen sich durch eine hohe Festigkeit sowie durch eine hohe Temperatur- und Oxidationsbeständigkeit aus. Diese Eigenschaften legen es nahe, die vergleichsweise teuren Metallschäume am Ende der Nutzungsdauer der Inhalatorkomponente 2 zu recyclen und wiederzuverwenden. Enthält das flüssige Material 18 Nikotin, sollte die Inhalatorkomponente 2 grundsätzlich nur gegen ein angemessenes Pfand an den Verbraucher abgegeben werden. Auf diese Weise wird sichergestellt, daß der Großteil der mit Nikotinrückständen kontaminierten Kühler 36, Schwämme 27a, 27b und Flüssigkeitsbehälter 19 umweltgerecht entsorgt und gegebenenfalls wiederaufbereitet wird.

Am Ende der Heizperiode deaktiviert der Schaltkreis 7 den Schalter 7a für ein paar Sekunden. Die Deaktivierung wird dem Benutzer zum Beispiel durch eine Leuchtdiode angezeigt und ist erforderlich, damit die Verbunde 10 abkühlen können, und die Dochte sich von neuem mit dem flüssigen Material 18 vollsaugen können. Der Flüssigkeitstransport wird ursprünglich durch die Kapillarität der Verbunde 10 bzw. deren Dochte induziert. Die Dochte saugen das flüssige Material 18 über die Verbund-Endabschnitte 10a, 10b aus den Kapillarspalt-Ästen 16a, 16b (siehe Fig. 4b und Fig. 10). Die Dochte werden also von zwei Seiten infiltriert. Die Entnahme von flüssigem Material 18 aus den Kapillarspalt-Ästen 16a, 16b induziert im Kapillarspalt 16 einen Kapillardruck, welcher bis in den Flüssigkeitsbehälter 19 zurückwirkt, wodurch flüssiges Material 18 aus dem Flüssigkeitsbehälter 19 über die Versorgungsöffnung 20 und die Durchtrittsöffnung 22 in den Kapillarspalt 16 nachströmen kann (siehe Pfeile in Fig. 4b). Die aus dem Flüssigkeitsbehälter 19 entnommene Menge flüssigen Materials 18 wird im Zuge eines Druckausgleichs durch eine äquivalente Menge Luft ersetzt. Der Druckausgleich erfolgt über die Belüftungsnut 25 und die Belüftungsöffnung 21. Sobald die Verbunde 10 bzw. Dochte vollständig mit dem flüssigen Material 18 infiltriert sind, steht der Inhalator für einen neuerlichen Verdampfungszyklus bereit.

Abschließend soll beispielhaft noch eine nikotinhaltige Zubereitung des flüssigen Materials 18 offenbart werden, welche in Prototypen verdampft wurde (siehe Tabelle 1). Das hierbei gebildete und verabreichte Kondensationsaerosol kam hinsichtlich der pharmakologischen, pharmakokinetischen sowie organoleptischen Wirkungen dem Rauch einer konventionellen Zigarette sehr nahe. Sämtliche aufgeführten Inhaltsstoffe finden sich auch im Zigarettenrauch wieder.

**Tabelle 1:**

| Stoff | CAS-Nummer | Massen-% |
|---|---|---|
| Wasser | 7732-18-5 | 52,92 |
| Ethanol | 64-17-5 | 3,80 |
| Glycerol (E422) | 56-81-5 | 40,10 |
| Nikotin | 54-11-5 | 1,60 |
| Milchsäure (E270) | 50-21-5 | 0,29 |
| Bernsteinsäure (E363) | 110-15-6 | 0,32 |
| Benzoesäure (E210) | 65-85-0 | 0,26 |
| Essigsäure (E260) | 64-19-7 | 0,71 |
| | Summe: | 100,00 |

Es sei noch darauf hingewiesen, daß die Erfindung selbstverständlich nicht auf einen oder mehrere flächige Verbunde 10 gemäß dem soeben beschriebenen Ausführungsbeispiel beschränkt ist. Die Verbunde 10 können ebenso linien- bzw. fadenförmig ausgebildet sein. Die Verbunde müssen auch nicht zwingend eben bzw. geradlinig sein, sondern können vielmehr jede beliebige Gestalt aufweisen. Die Verbunde können ferner in beliebiger Weise miteinander elektrisch verschaltet sein. Schließlich umfaßt die Erfindung auch Vorrichtungen, in welchen der Flüssigkeitsbehälter 19 vom Gehäuse 3 trennbar angeordnet ist, so daß der Flüssigkeitsbehälter 19, sobald er leer ist, durch einen neuen Flüssigkeitsbehälter ersetzt werden kann.

### Bezugszeichenliste

- 1: wiederverwendbares Inhalatorteil
- 2: auswechselbare Inhalatorkomponente
- 3: Gehäuse
- 4: Mundstück
- 5: Hauptgehäuse
- 6: Batterie
- 7: elektrischer Schaltkreis
- 7a: Schalter
- 8a: Steckzungen
- 8b: Steckbuchsen
- 9a: Führungsnasen
- 9b: Führungsnuten
- 10: flächige Verbunde
- 10a, 10b: Verbund-Endabschnitte
- 11: Trägerplatte, Leiterplatte, Multilayer-Leiterplatte
- 11a: Leiterplatten-Vorderseite
- 11b: Leiterplatten-Rückseite
- 12: Ausnehmung
- 13: Leiterbahnen
- 14: Oberteil
- 14a, 14b: Vorsprünge
- 15: Aussparung
- 16: Kapillarspalt
- 16a, 16b: Kapillarspalt-Äste
- 17: Haltewinkel
- 18: flüssiges Material
- 19: Flüssigkeitsbehälter
- 20: Versorgungsöffnung
- 21: Belüftungsöffnung
- 22: Durchtrittsöffnung
- 23: Fortsatz
- 24: Steg
- 25: Belüftungsnut
- 26: Luftvolumen, Luftpolster
- 27a, 27b: offenporige, saugfähige Schwämme
- 28: Kammer
- 29: U-förmiger Träger
- 29a, 29b: Winkelprofile
- 29c: Schenkel
- 30: Vertiefung
- 31: Luftdüse
- 32: Ansaugschnorchel
- 33: Loch
- 34: Strömungsdrossel
- 35: Plenumkammer
- 36: Kühler

## Patentansprüche

1. Inhalatorkomponente für die Bildung eines Dampf-Luft-Gemisches oder/und Kondensationsaerosols durch Verdampfung eines flüssigen Materials (18) und gegebenenfalls Kondensation des gebildeten Dampfes, umfassend:
ein elektrisches Heizelement zur Verdampfung einer Portion des flüssigen Materials (18);
einen Docht mit einer Kapillarstruktur, welcher Docht mit dem Heizelement einen Verbund (10) bildet und das Heizelement selbsttätig mit dem flüssigen Material (18) versorgt;
eine Trägerplatte (11), vorzugsweise Leiterplatte, welche den Verbund (10) trägt und auf welcher das Heizelement elektrisch kontaktiert ist;
einen zumindest zum Teil durch die Trägerplatte (11) gebildeten Kapillarspalt (16) zur selbsttätigen Versorgung des Verbundes (10) mit dem flüssigen Material (18), indem ein Endabschnitt des Dochts in den Kapillarspalt (16) ragt;
einen das flüssige Material (18) enthaltenden Flüssigkeitsbehälter (19), von welchem der Kapillarspalt (16) das flüssige Material (18) bezieht,
**dadurch gekennzeichnet, daß** der Kapillarspalt (16) den Flüssigkeitsbehälter (19) in einer Ansicht senkrecht zur Trägerplatte (11) außen zumindest teilweise überdeckt.

2. Inhalatorkomponente **nach Anspruch 1, dadurch gekennzeichnet, daß** der Verbund (10) den Flüssigkeitsbehälter (19) in einer Ansicht senkrecht zur Trägerplatte (11) zumindest teilweise überdeckt.

3. Inhalatorkomponente **nach Anspruch 1 oder 2, dadurch gekennzeichnet, daß** die Trägerplatte (11) zumindest abschnittsweise auf dem Flüssigkeitsbehälter (19) lagert.

4. Inhalatorkomponente **nach Anspruch** 3, **dadurch gekennzeichnet, daß** der Flüssigkeitsbehälter (19) im Wesentlichen die Form eines Quaders hat, und die Trägerplatte (11) zumindest abschnittsweise auf einer Seitenfläche des Quaders lagert.

5. Inhalator, umfassend eine Inhalatorkomponente (2) **nach einem der Ansprüche 1-4.**

## Claims

1. Inhaler component for forming a vapour/air mixture or/and condensation aerosol by evaporation of a liquid material (18) and, if appropriate, condensation of the formed vapour, comprising:
an electric heating element for evaporating a portion of the liquid material (18);
a wick with a capillary structure, which wick forms a composite (10) with the heating element and supplies the heating element automatically with the liquid material (18);
a carrier plate (11), preferably a printed circuit board, which carries the composite (10) and on which the heating element is electrically contacted;
a capillary gap (16) formed at least partially by the carrier plate (11) and automatically supplying the composite (10) with the liquid material (18), by means of an end portion of the wick extending into the capillary gap (16);
a liquid container (19) which contains the liquid material (18) and from which the capillary gap (16) draws the liquid material (18),
**characterized in that** the capillary gap (16) at least partially covers the liquid container (19) on the outside, in a view perpendicular to the carrier plate (11).

2. Inhaler component according to Claim 1, **characterized in that** the composite (10) at least partially covers the liquid container (19), in a view perpendicular to the carrier plate (11).

3. Inhaler component according to Claim 1 or 2, **characterized in that** the carrier plate (11) bears at least in part on the liquid container (19).

4. Inhaler component according to Claim 3, **characterized in that** the liquid container (19) has substantially the shape of a cuboid, and the carrier plate (11) bears at least in part on a side surface of the cuboid.

5. Inhaler comprising an inhaler component (2) according to one of Claims 1-4.

## Revendications

1. Composant d'inhalateur pour la formation d'un mélange de vapeur et d'air et/ou d'un aérosol de condensation par évaporation d'une matière fluide (18) et éventuellement condensation de la vapeur formée, comprenant :
un élément chauffant électrique pour l'évaporation d'une portion de la matière fluide (18) ;
une mèche avec une structure capillaire, laquelle mèche forme avec l'élément chauffant un composite (10) et alimente l'élément chauffant automatiquement avec la matière fluide (18) ;
une plaque de support (11), de préférence une carte à circuits imprimés, qui porte le composite (10) et sur laquelle l'élément chauffant est mis en contact électrique ;
une fente capillaire (16) formée au moins en partie par la plaque de support (11) pour l'alimentation automatique du composite (10) avec la matière fluide (18), en ce qu'une portion d'extrémité de la mèche pénètre dans la fente capillaire (16) ;
un récipient de liquide (19) contenant la matière fluide (18), depuis lequel la fente capillaire (16) reçoit la matière fluide (18),
**caractérisé en ce que** la fente capillaire (16) recouvre au moins partiellement à l'extérieur le récipient de liquide (19) dans une vue perpendiculaire à la plaque de support (11).

2. Composant d'inhalateur selon la revendication 1, **caractérisé en ce que** le composite (10) recouvre le récipient de liquide (19) au moins en partie dans une vue perpendiculaire à la plaque de support (11).

3. Composant d'inhalateur selon la revendication 1 ou 2, **caractérisé en ce que** la plaque de support (11) s'appuie au moins en partie sur le récipient de liquide (19).

4. Composant d'inhalateur selon la revendication 3, **caractérisé en ce que** le récipient de liquide (19) présente essentiellement la forme d'un quadrilatère et la plaque de support (11) s'appuie au moins en partie sur une surface latérale du quadrilatère.

5. Inhalateur comprenant un composant d'inhalateur (2) selon l'une quelconque des revendications 1 à 4.
